# DEMANDE DE BREVET EUROPEEN

(11) **EP 0 855 593 A1**
(43) Date de publication de la demande: **29.07.1998**
(21) Numéro de dépôt: 97200161.4
(22) Date de dépôt: 20.01.1997
(51) Int. Cl.: G01N 21/35, C12Q 1/22

(54) **Procédé de détermination de la stérilité d'un échantillon**

(71) Demandeur: SOCIETE DES PRODUITS NESTLE S.A., 1800 Vevey (CH)
(72) Inventeur: Eyer, Kurt, 3600 Thun (CH); Bürgin, Daniela, 3322 Schoenbuehl (CH); Hill, Georges, 3007 Berne (CH); Neumann, Fred, 3612 Steffisburg (CH)
(74) Mandataire: Thomas, Alain

(57) **Abrégé**

L'invention concerne un procédé de détermination de la stérilité ou de la non-stérilité d'un échantillon disposé dans un emballage, dans lequel on fait des spectres dans le domaine de l'infra-rouge proche pour un produit donné sur des échantillons stériles et des échantillons non stériles, de manière à définir pour chaque spectre un point dans un système de coordonnées multi-dimensionnels appelé analyse principale de produit et réalisant ainsi une zone qu'on appelle zone stérile C et une zone qu'on appelle zone non stérile D et dans lequel on effectue ensuite un spectre dans le même domaine de l'infra-rouge proche pour le produit à analyser ainsi que son analyse principale de produit, de manière à voir si ledit point d'analyse tombe dans la zone stérile ou dans la zone non stérile.

## Description

L'invention concerne un procédé de détermination de la stérilité ou de la non-stérilité d'un échantillon disposé dans un emballage, ledit échantillon étant un produit liquide, semi-liquide ou visqueux.

Il existe déjà des procédés et des dispositifs permettant de détecter la stérilité voire l'absence de stérilité dans un emballage. Le brevet US 5'164'597 concerne un dispositif permettant de déterminer la stérilité d'un échantillon, dans lequel on fait un spectre dans l'infra-rouge proche de l'échantillon à étudier et on compare ce spectre avec des spectres de référence effectués sur des échantillons stériles et non-stériles. Le problème de ce type de dispositif est qu'il comprend une lumière incidente multi-faisceaux avec un miroir, ce qui conduit à un dispositif peu robuste et qui se dérègle facilement. D'autre part, le fait de faire différents spectres et de les comparer ne conduit pas à un procédé très fiable et très sensible.

Le but de la présente invention est de mettre au point un procédé de détermination de la stérilité d'un échantillon basé également sur une mesure dans l'infra-rouge proche, mais avec un dispositif plus robuste et ledit procédé donnant des résultats avec plus de garantie.

L'invention concerne un procédé de détermination de la stérilité ou de la non-stérilité d'un échantillon disposé dans un emballage, ledit échantillon étant un produit liquide, semi-liquide ou visqueux, dans lequel on fait des spectres dans le domaine de l'infra-rouge proche pour un produit donné sur des échantillons stériles et des échantillons non stériles, de manière à définir pour chaque spectre un point dans un système de coordonnées multi-dimensionnels appelé analyse principale de produit et réalisant ainsi une zone qu'on appelle zone de produit stérile et une zone qu'on appelle zone de produit non stérile et dans lequel on effectue ensuite un spectre dans le même domaine de l'infra-rouge proche pour le produit à analyser ainsi que son analyse principale de produit, de manière à voir si ledit point d'analyse tombe dans la zone stérile ou la zone non stérile.

Le principe de mesure selon l'invention dans l'infra-rouge proche est basé sur la mesure de la réflexion d'un faisceau incident tombant sur l'échantillon à étudier en faisant varier la longueur d'onde, dans le cas présent entre 10000 et 4000 cm-1. La quantité de lumière réfléchie est fonction du produit analysé. On réalise ainsi pour chaque échantillon stérile et non-stérile son spectre photométrique et une intégration de chaque spectre grâce à un logiciel spécifique basé sur certaines données caractéristiques donne une analyse principale de produit. Les différents point obtenus pour un échantillon stérile donnent une zone de produit stérile de même que les différents points obtenus pour un échantillon non stérile donnent une zone de produit non stérile. Le grand avantage de cette solution est qu'elle permet de définir des zones bien précises et bien séparées : de sorte que pour le nouvel échantillon à étudier la stérilité voire la non-stérilité ressortent de manière certaine.

Le type de photomètre utilisé pour faire les spectres infra-rouge proche n'est pas critique. On utilise de préférence un spectrophotomètre de la Société Bühler, à savoir le dispositif appelé "NIRVIS" ( marque déposée). Cet appareil est utilisé tel quel.

La mesure en tant que telle avec l'appareil utilisé est effectué en une durée très brève, de l'ordre de 15 secondes. Il faut bien sûr au préalable faire un étalonnage de l'appareil pour définir les zones de stérilité et non-stérilité. Sur le type de produit à analyser, on fait les différents spectres avec le produit stérile. Pour faire les spectres sur des échantillons non stériles, on injecte sur le produit à analyser différents microorganismes comme par exemple Pseudomonas spp, Micrococci spp, Bacillus spp, des levures et des moisissures ainsi que Staphylococcus aureus qu'on laisse incuber pendant quelques jours avant de faire le spectre photométrique. Avec l'appareil utilisé on arrive à une limite de détection de 10³ CFU/ml.

Les spectres obtenus dépendent de la température à laquelle on travaille. On préfère travailler à une température de l'ordre de 30 °C.

Il est possible selon l'invention de travailler avec la sonde du spectrophotomètre directement sur l'emballage, s'il est transparent à l'infra-rouge proche, par exemple sur une poche souple contenant une solution de produit pour alimentation entérale ou parentérale: il s'agit dans ce cas d'une analyse non destructive. Il est également possible de travailler en analyse destructive, à savoir avec un emballage opaque à l'infra-rouge proche, nécessitant une ouverture de l'emballage pour effectuer la mesure.Dans ce cas, après ouverture de l'emballage, on y introduit la sonde et on effectue la mesure in situ.

Chaque fois qu'on démarre une mesure sur un nouveau produit, on en fait un étalonnage pour fixer les zones de stérilité voire de non stérilité et on peut alors garder en mémoire cet étalonnage et l'utiliser chaque fois qu'on fera une mesure sur ce type d'échantillon.

Il est également possible de faire des mesures sur des produits contenant des particules en suspension, comme du lait au chocolat. Dans ce cas, il faut faire une homogénéisation du produit avant la mesure, de manière à avoir une distribution uniforme des particules dans la masse du produit.

Comme déjà mentionné ci-dessus, le spectrophotomètre utilisé est connu dans sa fonctionnalité d'obtention d'un spectre en infra-rouge proche pour la caractérisation d'un produit analysé. La présente invention tire partie de cette possibilité de faire des spectres, pour en tirer, grâce à un logiciel d'intégration du spectre, des conclusions s'agissant de la stérilité voire de la non-stérilité d'un échantillon.

L'invention concerne donc également l'utilisation d'un appareil mono-faisceau d'analyse dans l'infra-rouge proche pour la détermination de la stérilité ou de la non-stérilité d'un échantillon.

La suite de la description est faite en référence aux dessins sur lesquels
Fig. 1 est un spectre de lait UHT,
Fig. 2 est la représentation de l'analyse principale de produit,
Fig. 3 donne des spectres à différentes températures,
Fig. 4 donne la représentation de l'analyse principale de produit correspondante,
Fig. 5 et 6 donnent des spectres sans et avec homogénéisation.

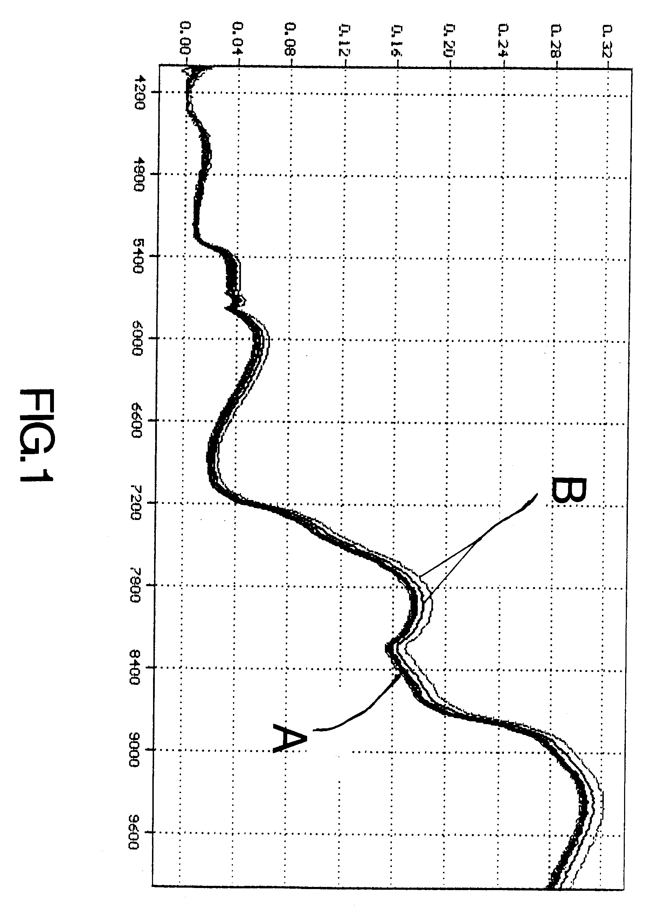

Des échantillons de lait UHT sont remplis dans des poches en plastique et on y injecte dans une moitié les microorganismes mentionnés ci-dessus et la seconde moitié reste en l'état. On les laisse incuber pendant quelques jours à 30 °C et on effectue ensuite un spectre IR proche avec un appareil Nirvis de la Société Bühler. La figure 1 représente le spectre avec en A la courbe obtenue pour l'échantillon stérile donc non infectée et en B la courbe pour l'échantillon infecté : l'abscisse donne la longueur d'onde et l'ordonnée la réflectance.

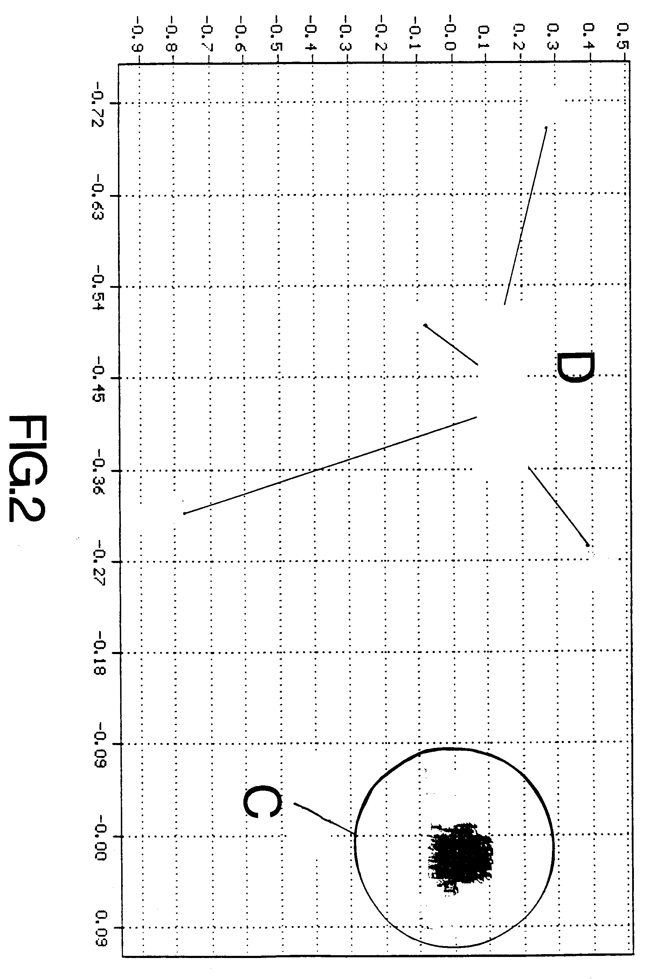

Pour bien être sûr de la qualité de la mesure, on choisit 100 échantillons non infectés et le même nombre d'échantillons infectés. Pour chacun de ces spectres, le système définit un point dans le système de coordonnées de la figure 2, à savoir une analyse principale de produit pour chaque spectre IR. On définit ainsi un ensemble de points C pour les échantillons stériles et un ensemble de points D pour les échantillons non stériles. Dans cette figure, l'abscisse et l'ordonnée représentent des paramètres mathématiques sans dimension.

L'étalonnage consiste donc à définir ces 2 zones C et D. Lorsqu'on a défini ces zones, on peut faire les tests de stérilité sur les échantillons de la même famille. Ces mesures sont très rapides, de l'ordre de 7 à 15 secondes et permettent de mettre en évidence une concentration de moins de 10³ CFU/ml.

On dispose ainsi à partir d'un appareil connu, d'une méthode sûre et rapide permettant de bien mettre en évidence la stérilité d'un produit dans un emballage en mesure destructive ou non destructive.

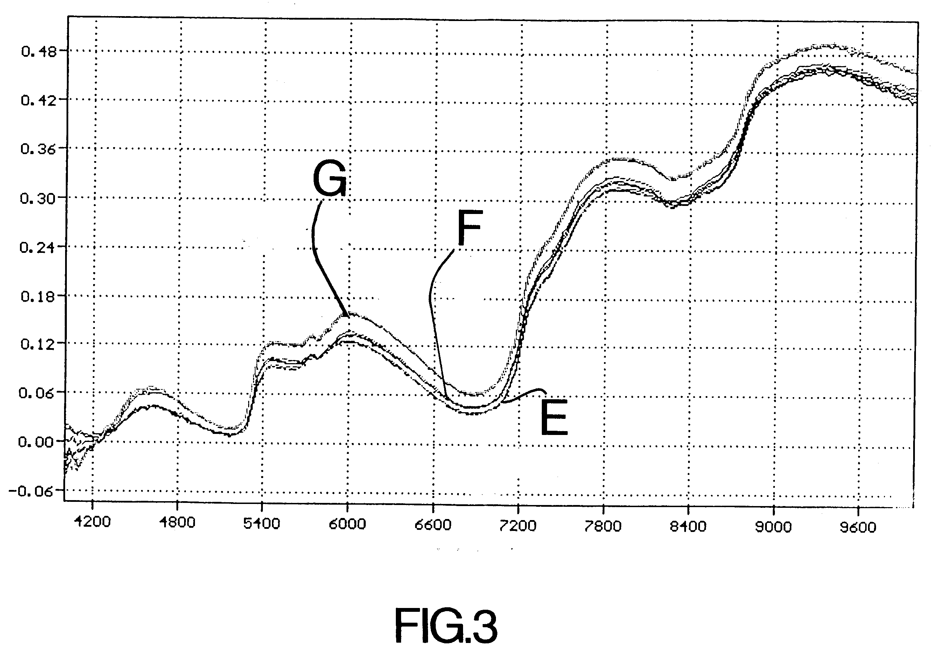

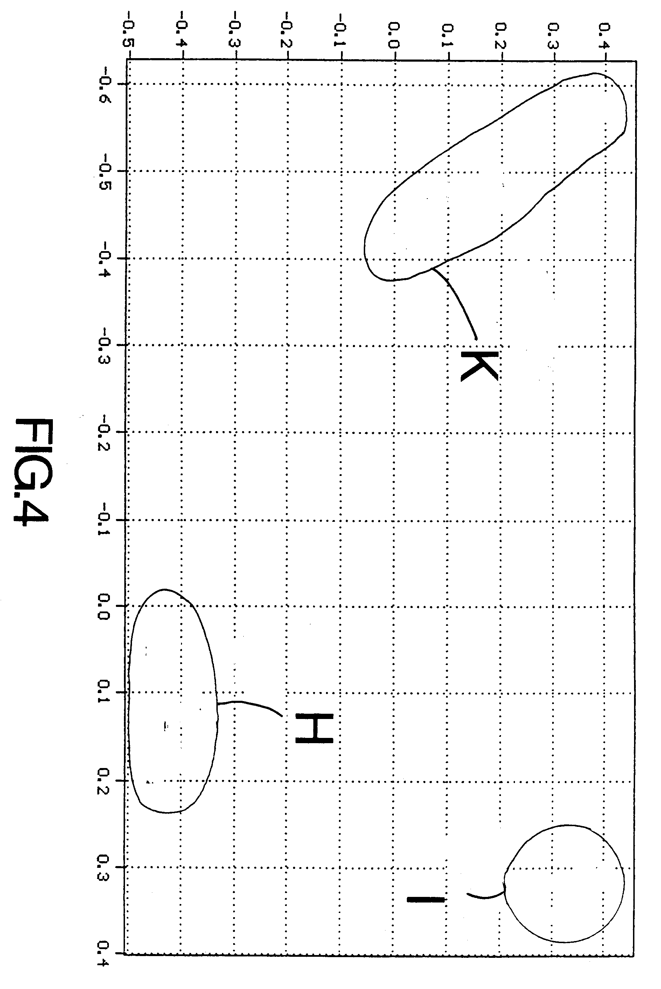

La figure 3 donne des spectres en infra-rouge proche pour le même produit stérile à différentes températures, à savoir la courbe E prise à 20°C, la courbe F à 30°C et la courbe G à 55°C. La figure 4 donne les zones de stérilité correspondantes pour ce produit, à savoir la zone I à 20°C, la zone H à 30°C et la zone K à 55°C. Il est à noter que l'on fait la mesure à la température à laquelle on a fait l'incubation.

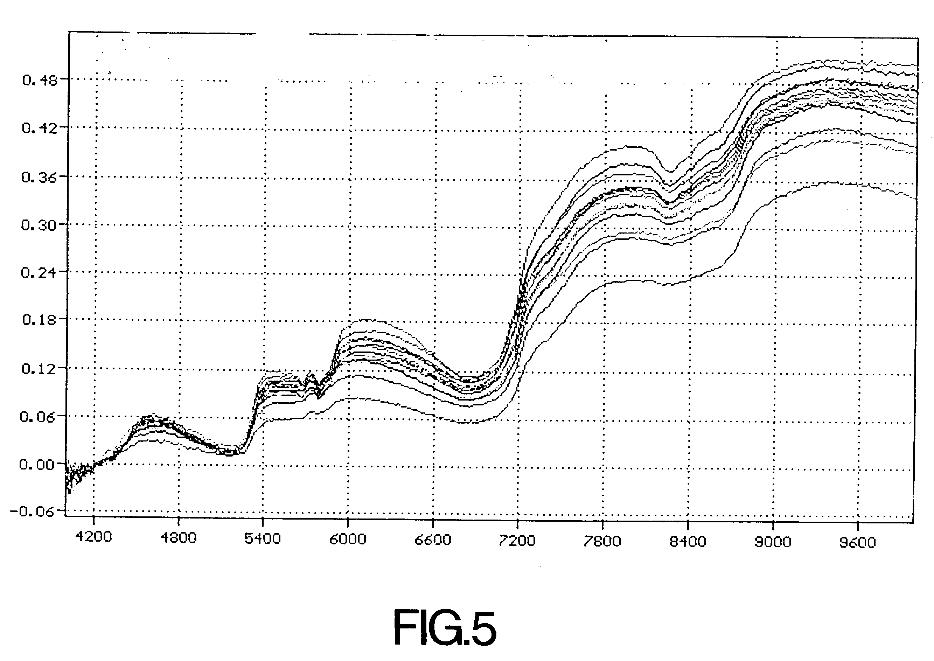

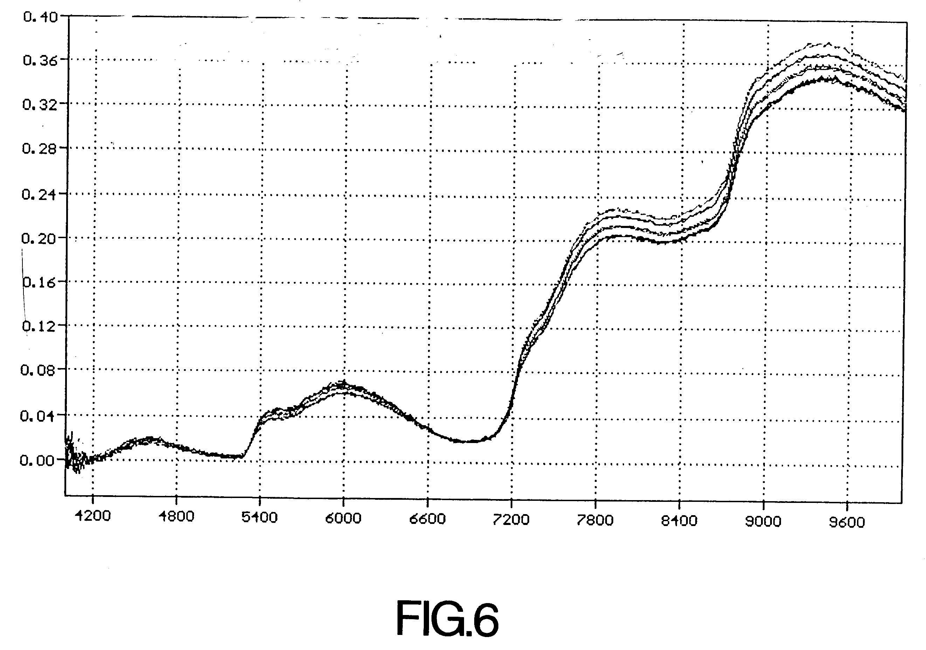

La figure 5 donne des spectres d'un produit n'ayant pas subi d'homogénéisation , alors que la figure 6 donne les spectres du même produit mais ayant subi l'homogénéisation.

## Revendications

1. Procédé de détermination de la stérilité ou de la non-stérilité d'un échantillon disposé dans un emballage,ledit échantillon étant un produit liquide, semi-liquide ou visqueux, dans lequel on fait des spectres dans le domaine de l'infra-rouge proche pour un produit donné sur des échantillons stériles et des échantillons non stériles, de manière à définir pour chaque spectre un point dans un système de coordonnées multi-dimensionnels appelé analyse principale de produit et réalisant ainsi une zone qu'on appelle zone stérile et une zone qu'on appelle zone non stérile et dans lequel on effectue ensuite un spectre dans le même domaine de l'infra-rouge proche pour le produit à analyser ainsi que son analyse principale de produit, de manière à voir si ledit point d'analyse tombe dans la zone stérile ou dans la zone non stérile.

2. Procédé selon la revendication 1, caractérisé en ce que le spectre est effectué entre 10000 et 4000 cm-1.

3. Procédé selon l'une des revendications 1 ou 2, caractérisé en ce qu'on opère sur l'emballage fermé ou l'emballage ouvert.

4. Procédé selon l'une des revendications 1 à 3, caractérisé en ce qu'on homogénéise le produit avant d'effectuer le spectre.

5. Utilisation d'un appareil mono-faisceau d'analyse dans l'infra-rouge proche pour la détermination de la stérilité ou de la non-stérilité d'un échantillon.
